# EUROPEAN PATENT APPLICATION

(11) **EP 3 626 867 A1**
(43) Date of publication of application: **25.03.2020**
(21) Application number: 18802284.2
(22) Date of filing: 13.04.2018
(51) Int. Cl.: D01F 4/00, A61L 17/08, A61L 27/24, A61L 27/52, C07K 14/78

(54) **FILAMENT AND PRODUCTION METHOD THEREFOR**

(30) Priority: 18.05.2017 JP 2017098931
(71) Applicant: National Agriculture and Food Research Organization, Tsukuba-shi, Ibaraki 305-8517 (JP); Saga University, Saga-shi, Saga 840-8502 (JP)
(72) Inventor: TAKEZAWA, Toshiaki, Tsukuba-shi Ibaraki 305-8602 (JP); AOKI, Shigehisa, Saga-shi Saga 840-8502 (JP); ENAIDA, Hiroshi, Saga-shi Saga 840-8502 (JP); MIYAZAKI, Ayumi, Tsukuba-shi Ibaraki 305-8602 (JP)
(74) Representative: V.O.
(86) International application number: PCT/JP2018/015488
(87) International publication number: WO 2018/211877

(57) **Abstract**

The present invention provides a production method of a filament for which a hydrogel having lower strength than a native collagen gel can be used as a raw material. The present invention provides a filament obtained by the above production method and having excellent strength. The production method of the filament includes a step A of irradiating columnar hydrogel with ultraviolet rays; step B of vitrifying the columnar hydrogel after irradiation with the ultraviolet rays to obtain a filamentous hydrogel dried body; and a step C of rehydrating the filamentous hydrogel dried body to obtain a filamentous vitrigel, in this order. The filament is formed of a vitrigel dried body, and to which a hydrophobic solvent is adhered or impregnated.

## Description

### TECHNICAL FIELD

The present invention relates to a filament and a production method thereof.

Priority is claimed on Japanese Patent Application No. 2017-098931, filed May 18, 2017, the content of which is incorporated herein by reference.

### BACKGROUND ART

The inventors of the present invention have developed an atelocollagen vitrigel membrane and demonstrated that damaged tissues such as skin, cornea, trachea, articular cartilage, tympanic membrane, and esophagus can be regenerated. In this process, it was found that the atelocollagen vitrigel membrane is a biocompatible material having an epithelialization promoting effect and a scar formation inhibiting effect in a wound part.

Under such a background, the atelocollagen vitrigel membrane useful for skin tissue regeneration has already been researched and developed as a medical device by pharmaceutical companies and the like. On the other hand, as allowable artificial dermis consisting of atelocollagen as a raw material in Japan, there are three products of INTEGRA (registered trademark) (produced by Integra LifeSciences Corporation.), TERUDERMIS (registered trademark) (produced by Terumo Corporation) and PELNAC (registered trademark) (produced by Gunze Limited). These kinds of artificial dermis are templates for dermis reconstruction composed of an atelocollagen sponge and a silicon membrane. From these facts, the atelocollagen vitrigel is expected to expand the application thereof in the field of regenerative medicine, and thus development has been demanded not only for a membrane type atelocollagen vitrigel but also for a filament type atelocollagen vitrigel.

On the other hand, the inventors of the present invention have developed a technique for processing a native collagen vitrigel into an optional shape such as a membrane, a filament, or a tube (refer to, for example, Patent Document 1).

### Citation List

### Patent Literature

[Patent Document 1] Japanese Unexamined Patent Application, First Publication No. 2007-204881

### DISCLOSURE OF INVENTION

However, since the atelocollagen gel has less strength than the native collagen gel, in the method disclosed in Patent Document 1, it is difficult to produce a filamentous vitrigel using a hydrogel such as atelocollagen having lower strength than the native collagen gel.

The present invention has been made in view of the above circumstance, and provides a production method of the filament for which the hydrogel having lower strength than the native collagen gel can be used as a raw material. Further, there is also provided a filament obtained by the above production method and having excellent strength.

### Solution to Problem

As a result of intensive studies to achieve the above object, the inventors of the present invention have found that a columnar hydrogel can be irradiated with ultraviolet rays, vitrified, and rehydrated to produce a filamentous atelocollagen vitrigel, and thereby the present invention has been completed.

That is, the present invention includes the following aspects.

A production method of a filament according to a first aspect of the present invention is a method including
a step A of irradiating a columnar hydrogel with ultraviolet rays;
a step B of vitrifying the columnar hydrogel after irradiation with the ultraviolet rays to obtain a filamentous hydrogel dried body; and
a step C of rehydrating the filamentous hydrogel dried body to obtain a filamentous vitrigel, comprising in this order.

The production method of the filament according to the first aspect may further include a step D of re- vitrifying the filamentous vitrigel to obtain a filamentous vitrigel dried body after the step C.

The production method of the filament according to the first aspect may further include a step of E of re-irradiating the filamentous vitrigel dried body with ultraviolet rays after the step D.

The production method of the filament according to the first aspect may further include a step F of adhering or impregnating a hydrophobic solvent to the filamentous vitrigel dried body re-irradiated with ultraviolet rays after the step E.

In the production method of the filament according to the first aspect, the hydrogel may be an atelocollagen gel.

In the production method of the filament according to the first aspect, the filament may be a tissue regeneration filament which can be used as a suture filament.

In the production method of the filament according to the first aspect, the filament may be a cell transplant carrier.

In the production method of the filament according to the first aspect, the filament may be a supplementary material of a conjunctival fistula.

In the production method of the filament according to the first aspect, the filament may be a peritonitis inhibitor or a peritoneal fibrosis inhibitor.

A filament according to a second embodiment of the present invention is formed of a vitrigel dried body, and to which a hydrophobic solvent is adhered or impregnated. A breaking strength of the filament may be 0.1 kgf or more.

The breaking strength of the filament may be 0.2 kgf or more.

The vitrigel dried body may be an atelocollagen vitrigel dried body.

The hydrophobic solvent may be a silicone oil.

The filament may be a tissue regeneration filament which can be used as a suture filament.

The filament may be a cell transplant carrier.

The filament may be a supplementary material of a conjunctival fistula.

The filament may be a peritonitis inhibitor or a peritoneal fibrosis inhibitor.

### Advantageous Effects of Invention

According to the above embodiment, it is possible to provide a production method of the filament for which the hydrogel having lower strength than the native collagen gel can be used as a raw material. Further, it is also possible to provide a filament obtained by the above production method and having excellent strength.

### BRIEF DESCRIPTION OF DRAWINGS

FIG. 1A is a graph showing a breaking strength in a filamentous atelocollagen vitrigel dried body 1 under each condition in Example 1.
FIG. 1B is a graph showing a breaking strength in a filamentous atelocollagen vitrigel dried body 2 under each condition in Example 1.
FIG. 1C is a graph showing a breaking strength of polyglactin suture filament VICRYL (registered trademark) under each condition in Example 1.
FIG. 2 is an image in which an incision of a mouse is sutured using the filamentous atelocollagen vitrigel dried body 1 infiltrated with a silicone oil in Example 2.
FIG. 3A is an image showing a result of hematoxylin-eosin (HE) staining of a tissue section of a suture part of a mouse using the filamentous atelocollagen vitrigel dried body I infiltrated with a silicone oil (infiltrated body 1) on day 7 of the suture in Example 2.
FIG. 3B is an image showing a result of HE staining of the tissue section of the suture part of the mouse using a polyglactin suture filament VICRYL (registered trademark) (dried body 3) on day 7 of the suture in Example 2.
FIG. 3C is an image showing a result of immunostaining the tissue section of the suture part of the mouse using the filamentous atelocollagen vitrigel dried body 1 infiltrated with a silicone oil (infiltrated body 1) on day 7 of the suture in Example 2, with an anti-α smooth muscle actin (a-SMA) antibody.
FIG. 3D is an image showing a result of immunostaining the tissue section of the suture part of the mouse using a polyglactin suture filament VICRYL (registered trademark) (dried body 3) on day 7 of the suture in Example 2, with an α-SMA antibody.
FIG. 4A is an image in which a porcine conjunctival fistula is blocked using a filamentous atelocollagen vitrigel dried body 2 as a supplementary material in Example 3.
FIG. 4B is an image in which the porcine conjunctival fistula is blocked using a plastic port which is the supplementary material in the related art in Example 3.
FIG. 5A is an image of a rehydrated filamentous atelocollagen vitrigel dried body 1 (rehydrated body 1) cultured with endothelial cells in Example 4.
FIG. 5B is an image of a rehydrated filamentous atelocollagen vitrigel dried body 1 (rehydrated body 1) cultured with fibroblasts in Example 4.
FIG. 5C is an image of a rehydrated polyglactin suture filament VICRYL (registered trademark) (rehydrated body 3) cultured with the fibroblasts in Example 4.
FIG. 6A is an image of filamentous atelocollagen vitrigel dried bodies (CXM 1 and CXM 5) used in Example 5.
FIG. 6B is a view showing a method of inserting the filamentous atelocollagen vitrigel dried body into a mouse abdominal cavity in Example 5.
FIG. 6C is an image showing a state of inserting the filamentous atelocollagen vitrigel dried body into a mouse abdominal cavity in Example 5.
FIG. 7A are images showing states of peritoneum of each group of mice opened on day 40 after induction of peritonitis in Example 5. The upper images are images showing the entire abdomen of the opened mouse, and the lower images are enlarged images of the peritoneum. The scale bar indicates 1 cm.
FIG. 7B are HE stained images of the tissue section of each group of mice on day 40 after the induction of peritonitis in Example 5. The upper images are the HE stained images of the parietal peritoneum, and the lower images are the HE stained images of the visceral peritoneum. The scale bar indicates 100 µm.
FIG. 7C are Azan stained images of the tissue section of parietal peritoneum of each group of mice on day 40 after the induction of peritonitis in Example 5. The scale bar indicates 200 µm.
FIG. 7D is a graph showing a thickness of a submethothelial connective tissue of fibrotic peritoneum of each group of mice on day 40 after the induction of peritonitis in Example 5.
FIG. 8A are images showing states of peritoneum of each group of mice opened on day 56 after the induction of peritonitis in Example 5. The upper images are images showing the entire abdomen of the opened mouse, and the lower images are enlarged images of the peritoneum. The scale bar indicates 1 cm.
FIG. 8B are HE stained images of the tissue section of each group of mice on day 56 after the induction of peritonitis in Example 5. The upper images are the HE stained images of the parietal peritoneum, and the lower images are the HE stained images of the visceral peritoneum. The scale bar indicates 1 00 µm.
FIG. 8C are Azan stained images of the tissue section of parietal peritoneum of each group of mice on day 56 after the induction of peritonitis in Example 5. The scale bar indicates 200 µm.
FIG. 8D is a graph showing a thickness of a submethothelial connective tissue of fibrotic peritoneum of each group of mice on day 56 after the induction of peritonitis in Example 5.
FIG. 9 is an immunostained image with various antibodies of the tissue sections of peritoneum of each group of mice on day 40 after the induction of peritonitis in Example 5. The scale bar of the immunostained image with an anti-cytokeratin AE1/AE3 (CKAE1/AE3) antibody, an anti-vimentin antibody, and an anti-N-cadherin antibody indicates 50 µm. The scale bar of the immunostained image with an anti-connective tissue growth factor (CTGF) antibody and anti-α-SMA antibody indicates 100 µm.
FIG. 10A is an immunostained image with various antibodies of the tissue sections of peritoneum of each group of mice on day 40 after the induction of peritonitis in Example 5. The scale bar indicates 50 µm.
FIG. 10B is a graph showing the number of CD45 positive cells in a submesothelial interstitial layer (SMIL) of each group of mice on day 40 after the induction of peritonitis in Example 5.
FIG. 10C is a graph showing the number of F4/80 positive cells in SMIL of each group of mice on day 40 after the induction of peritonitis in Example 5.
FIG. 10D is a graph showing a proportion of proliferating cell nuclear antigen (PCNA) positive cells in the SMIL of each group of mice on day 40 after the induction of peritonitis in Example 5.

### BEST MODE FOR CARRYING OUT THE INVENTION

### <<Production method of the filament>>

### <First embodiment>

A production method of a filament according to a first embodiment of the present invention is a method including
a step A of irradiating a columnar hydrogel with ultraviolet rays;
a step B of vitrifying the columnar hydrogel after irradiation with the ultraviolet rays to obtain a filamentous hydrogel dried body; and
a step C of rehydrating the filamentous hydrogel dried body to obtain a filamentous vitrigel, comprising in this order.

In the production method in the related art, it was not possible to produce a filament using a hydrogel having a lower strength than a native collagen gel.
In contrast, according to the production method of the present embodiment, it is possible to produce the filament for which the hydrogel having lower strength than the native collagen gel as a raw material can be used.

Hereinafter, the details of each step of the production method of the filament according to the present embodiment will be described.

### [Step A]

First, a columnar hydrogel is irradiated with ultraviolet rays to enhance the strength of the columnar hydrogel.

The columnar hydrogel used in the step A may be any one obtained by, for example, using a cylindrical mold or the like so that a sol has a desired diameter. Moreover, the temperature which heat-retains the sol at the time of gelation may be appropriately adjusted depending on the kind of sol to be used. For example, in a case where the sol is a collagen sol, the heat retention during gelation may be at a temperature lower than a denaturation temperature of collagen depending on the animal species of collagen to be used, and generally, gelation can be performed in several minutes to several hours by the heat retention at the temperature of 20°C or higher and 37°C or lower.

In the present specification, "sol" means that a dispersoid colloidal particle (size: about 1 to several hundred nm) using a liquid as a dispersion medium is particularly formed of a polymer compound. More specifically, the sol is an aqueous solution of a natural product polymer compound or a synthetic polymer compound. Therefore, in a case where crosslinks are introduced by chemical bonds of these polymer compounds and take a mesh structure, the aqueous solution transforms into a "hydrogel", a semisolid substance with a large amount of water in the mesh. That is, "hydrogel" means a gelled sol.

In addition, "vitrigel" refers to a gel in a stable state obtained by vitrifying and rehydrating the hydrogel in the related art, and has been named "Vitrigel (registered trademark)" by the inventor".

Further, in the present specification, in describing the production steps of the present embodiment in detail, a dried body of the hydrogel immediately after the vitrifying step and not subjected to the rehydrating step was simply referred to as a "hydrogel dried body". In addition, the gel obtained through the rehydrating step after the vitrifying step was distinguished and represented as "vitrigel", and the dried body obtained by vitrifying the vitrigel was referred to as a "vitrigel dried body". Moreover, a substance obtained through a step of irradiating the vitrigel dried body with ultraviolet rays was referred to as a "filamentous vitrigel dried body re-irradiated with ultraviolet rays". Therefore, the "vitrigel" is a hydrate.

In addition, the sol used as a raw material for the columnar hydrogel may be any material having biocompatibility, and examples thereof include a component derived from an extracellular matrix available for gelation, natural polymer compounds such as fibrin, agar, agarose, and cellulose, and synthetic polymer compounds such as polyacrylamide, polyvinyl alcohol, polyethylene oxide and poly (II-hydroxyethyl methacrylate)/polycaprolactone.

Examples of the component derived from an extracellular matrix available for gelation include collagens (type I, type II, type III, type V, type XI, and the like), a reconstituted basement membrane component (trade name: MATRIGEL) derived from mouse EHS tumor extracts (including type IV collagen, laminin, heparan sulfate proteoglycan, and the like), glycosaminoglycan, hyaluronic acid, proteoglycan, and gelatin, and the examples are not limited to these. It is possible to produce a desired vitrigel by selecting components and the like of a salt, concentration, pH, and the like that are optimal for each gelation. In addition, by combining raw materials, it is possible to obtain vitrigels that mimic various in vivo tissues.

Among them, as the sol, the component derived from an extracellular matrix available for gelation is preferable, and the collagen is more preferable. In addition, a native collagen or an atelocollagen can be exemplified as a more preferable raw material among the collagens, and the atelocollagen is more preferable. That is, as the hydrogel used in the present steps, an atelocollagen gel is particularly preferable because it is a biocompatible material having an epithelialization promoting effect and a scar formation inhibiting effect in a wound part.

Further, depending on the composition and content, the hydrogel obtained from the sol exemplified above is a gel having the same strength as that of the native collagen gel, or a gel having lower strength than that of the native collagen gel. In addition, it may be a gel having higher strength than that of the native collagen gel. According to the production method of the present embodiment, even with the hydrogel with any of strength, it is possible to produce a filament by using the hydrogel obtained by the above exemplar examples of sol, as a raw material.

Note that, in the present specification, the "epithelialization promoting effect" means that the formation of regenerative epithelium is promoted by inhibiting the formation of granulation tissue and promoting the proliferation of epidermal cells in a healing process of the wound part. By using a filament made of atelocollagen vitrigel dried body as a suture filament, epithelialization is promoted, so that the wound can be healed in a shorter period of time than before.

Moreover, the "scar formation inhibiting effect" means an effect of inhibiting the formation of a so-called scar (a scar) remaining after the wound has healed. By using a filament made of atelocollagen vitrigel dried body as a suture filament, the wound part can be treated without leaving a scar.

For example, in a case where the hydrogel is vitrified in the following step B in a state where the columnar hydrogel is suspended, the irradiation energy of ultraviolet rays to the columnar hydrogel in this process is sufficient to have the strength such that the hydrogel is not cut, and may be appropriately adjusted according to the composition and content of the hydrogel. The irradiation energy of ultraviolet rays to the columnar hydrogel may be, for example, 0.1 mJ/cm² to 6,000 mJ/cm², for example, 10 mJ/cm² to 4,000 mJ/cm², and for example, 100 mJ/cm² to 3,000 mJ/cm².

In addition, the irradiation of the columnar hydrogel with ultraviolet rays is performed so that an ultraviolet irradiation region is divided into one surface and the other surface of the columnar hydrogel (for example, "upper surface and lower surface", or "adverse side and reverse side", or the like) so that the entire surface of the columnar hydrogel is irradiated with ultraviolet rays. The total irradiation amount may be set as the total irradiation amount of ultraviolet rays per unit area to the columnar hydrogel.

### [Step B]

Next, the columnar hydrogel after irradiation with the ultraviolet rays is dried and vitrified to obtain a filamentous hydrogel dried body.

By drying the columnar hydrogel, free water in the columnar hydrogel can be completely removed, and further partial removal of the bound water can proceed.

As the period of the vitrification step (step of proceeding with partial removal of bound water after completely removing the free water in the columnar hydrogel) is lengthened, a filamentous vitrigel excellent in transparency and strength can be obtained when rehydrated. Note that, if necessary, the filamentous vitrigel obtained by rehydration after vitrification for a short period of time can be washed with PBS or the like and vitrified again.

As a drying method, for example, various methods such as air drying, drying in a sealed container (circulating air in the container and always supplying dry air), drying in an environment where silica gel is placed, and the like can be used. For example, examples of the air drying method include a method of drying in an incubator kept sterile at 10°C and 40% humidity for two days, or drying at room temperature all day and night in a clean bench.

Further, in this step, since the columnar hydrogel has an appropriate strength by being irradiated with the ultraviolet rays in the above-mentioned step A, it can be efficiently dried in a state of being hung on a clothesline or the like during drying.

### [Step C]

Next, a filamentous vitrigel is obtained by rehydrating the obtained filamentous hydrogel dried body.

At this time, rehydration may be performed using saline, PBS (Phosphate Buffered Saline), or the like.

### <Second embodiment>

The production method of the filament according to a second embodiment of the present invention is a method of providing a step D of re-vitrifying the filamentous vitrigel to obtain a filamentous vitrigel dried body after the step C.

According to the production method of the present embodiment, it is possible to produce the filament for which the hydrogel having lower strength than the native collagen gel as a raw material can be used.

Steps A to C are as described in the first embodiment. Details of step D in the present embodiment will be described below.

### [Step D]

Next, the obtained filamentous vitrigel is dried and vitrified again.

Examples of the drying method include the same methods as those exemplified in Step B above.

Moreover, in this process, since the filamentous vitrigel has an appropriate strength, it can be efficiently dried while being hung on a clothesline or the like.

### <Third embodiment>

The production method of the filament according to a third embodiment of the present invention is a method further including a step E of re-irradiating the filamentous vitrigel dried body with ultraviolet rays after the step D.

According to the production method of the present embodiment, it is possible to produce the filament for which the hydrogel having lower strength than the native collagen gel as a raw material can be used. Further, the filament obtained by the production method of the present embodiment has excellent strength.

The step A to step D are as described in the first embodiment and second embodiment. Details of step E in the present embodiment will be described below.

### [Step E]

Next, obtained filamentous vitrigel dried body is re-irradiated with ultraviolet rays to further increase the strength of the filamentous vitrigel dried body.

The irradiation energy of the ultraviolet rays to the filamentous vitrigel dried body in this step may be a strength that is not cut in a case where the filamentous vitrigel dried body is used as a suture filament or the like, and may be appropriately adjusted according to the composition and content of the filamentous vitrigel dried body. The irradiation energy of ultraviolet rays to the filamentous vitrigel dried body may be, for example, 0.1 mJ/cm² to 6,000 mJ/cm², for example, 10 mJ/cm² to 4,000 mJ/cm², and for example, 100 mJ/cm² to 3,000 mJ/cm².

In addition, the irradiation of the filamentous vitrigel dried body with ultraviolet rays is performed so that an ultraviolet irradiation region is divided into one surface and the other surface of the filamentous vitrigel dried body (for example, "upper surface and lower surface", or "adverse side and reverse side", or the like) so that the entire surface of the filamentous vitrigel dried body is irradiated with ultraviolet rays. The total irradiation amount may be set as the total irradiation amount of ultraviolet rays per unit area to the filamentous vitrigel dried body.

### <Fourth embodiment>

The production method of the filament according to a fourth embodiment of the present invention is a method including a step F of adhering or impregnating a hydrophobic solvent to the filamentous vitrigel dried body re-irradiated with ultraviolet rays after the step E.

According to the production method of the present embodiment, it is possible to produce the filament for which the hydrogel having lower strength than the native collagen gel as a raw material can be used. Further, the filament obtained by the production method of the present embodiment has increased flexibility due to the hydrophobic solvent acting as a lubricant, and includes the hydrophobic solvent, so that the strength can be maintained even in the presence of an aqueous solvent.

The step A to step E are as described in the first embodiment, the second embodiment and the third embodiment. Details of step F in the present embodiment will be described below.

### [Step F]

Next, a hydrophobic solvent is adhered to or impregnated into the filamentous vitrigel dried body after being re-irradiated with the ultraviolet rays. With this, the flexibility of the filamentous vitrigel dried body is increased, and when used in applications such as a suture filament or the like, the hydrophobic solvent acts as a lubricant, and a sutured part can be smoothly sutured without being caught. In addition, by adhering the hydrophobic solvent onto the surface of the filamentous vitrigel dried body or impregnating the hydrophobic solvent into the inside from directly under the surface, the deterioration of the strength due to the rehydration by a body fluid or the like is prevented at the suture part, and thereby the strength of the filament can be maintained.

Here, "adhere" means that the hydrophobic solvent is adhered to the surface of the filamentous vitrigel dried body, and "impregnate" means that the hydrophobic solvent is soaked into the filamentous vitrigel dried body directly from the surface thereof.

The hydrophobic solvent used in this step is not particularly limited as long as it has biocompatibility, and examples thereof include hydrophobic solvents used in known medical applications. Specific examples of the hydrophobic solvent include medical paraffin; liquid oils from plant, seafood, or animal sources (for example, olive oil, corn oil, soybean oil, canola oil, cottonseed oil, coconut oil, sesame oil, sunflower oil, borage seed oil, clove oil, hemp seed oil, herring oil, cod liver oil, salmon oil, linseed oil, malt oil, and Evening primrose oil, and mixtures thereof in any proportions); polyunsaturated oils containing ω-3 and ω-6 fatty acids such as linoleic and linolenic acid, γ-tinoleic acid (GLA), eicosapentaenoic acid (EPA), and docosahexaenoic acid (DHA); essential oils derived from rose, canoki, burdock, camphor, cardamom, carrot, kousu igaya, onisalvia (clary), sage, clove, cypress, frankincense, ginger, grapefruit, hyssop, jasmine, lavender, lemon, mandarin, majorana, pills, neroli oil, nutmeg, petitgrain, sage, tangerine, vanilla, verbena, and the like; a triglyceride oil; and a silicone oil. However, the hydrophobic solvent is not limited to these examples. These hydrophobic solvents may be used alone or a combination thereof may be used.

Among these, the hydrophobic solvent is preferably a silicone oil. The silicone oil may be unmodified or modified. More specific examples of the silicone oil include, but are not limited to, polydimethyl siloxane, polymethyl phenyl siloxane, polydiphenyl siloxane, and polymethyl hydrogen siloxane, and the like.

The temperature for adhering or impregnating the hydrophobic solvent is not particularly limited, and may be, for example, 10°C or higher and 40°C or lower.

The time for adhering or impregnating the hydrophobic solvent is not particularly limited, and may be, for example, 30 minutes to 48 hours.

Examples of the method of adhering or impregnating the hydrophobic solvent include a method of immersing the hydrophobic solvent to the filamentous vitrigel dried body, a method of applying the hydrophobic solvent to the filamentous vitrigel dried body, and a method of spraying the hydrophobic solvent to the filamentous vitrigel dried body, and the examples are not limited to these methods.

### <Application>

As will be described in examples below, the filament obtained by the production method of the present embodiment can be used as, for example, a tissue regeneration filament and a cell transplant carrier. Alternatively, as will be described in examples below, the filament obtained by the production method of the present embodiment can be used as a supplementary material of conjunctival fistula to be used for inserting a treatment device into the vitreous body during a vitreous surgery. Alternatively, as will be described in examples below, the filament obtained by the production method of the present embodiment can be used as an indwelling material which inhibits peritonitis and peritoneal fibrosis by being inserted into the abdominal cavity.

### <<Filament>>

A filament according to one embodiment of the present invention is formed of a vitrigel dried body, and to which a hydrophobic solvent is adhered or impregnated.

Although the filament according to the present embodiment is formed of a vitrigel dried body, it has excellent strength and can be used as a suture filament or the like. Further, the filament according to the present embodiment has increased flexibility due to the hydrophobic solvent acting as a lubricant, and includes the hydrophobic solvent, so that the strength can be maintained even in the presence of an aqueous solvent. Therefore, in a case where the filament according to the present embodiment is used as a suture filament, the suture part is maintained without being released after the operation.

### <Physical properties>

The shape of the cross section of the filament according to the present embodiment is not particularly limited, and examples thereof include polygons such as triangle, square (including square, rectangle, and trapezoid), pentagon, hexagon, heptagon, and octagon; a circular shape, an elliptical shape, an approximately circular shape, an elliptical shape, an approximately elliptical shape, a semicircular shape, and a fan shape. Among these, it is preferable that the shape of the cross section of the filament is circular.

Moreover, in a case where the shape of the cross section of the filament according to the present embodiment is a circular shape, the average diameter may be suitably selected in accordance with the applications. The average diameter of the filament may be, for example, 1 µm to 1 mm, and for example, 3 µm to 900 µm.

The breaking strength of the filament according to the present embodiment may be a strength that does not cut in a case of being used as a suture filament or the like. Specifically, the breaking strength of the filament may be, for example, 0.1 kgf or more, and may be, for example, 0.2 kgf or more. When the lower limit of the breaking strength of the filament is equal to or more than the above range, the filament can be strong enough not to cut in a case of being used as a suture filament or the like.

The method for measuring the breaking strength of the filament is as follows.

After cutting the filament to a total length of 5 cm, and the filament pulled at 9 mm per minute while fixing both ends at 1 cm, and the breaking strength (kgf) at this time may be measured by using a digital force gauge (manufactured by Nidec-Shimpo Corporation).

Next, the structural components of the filament according to the present embodiment will be described in detail below.

### <Vitrigel dried body>

The filament according to the present embodiment consists of vitrigel dried body.

Examples of the sol that is a raw material for the vitrigel dried body include the same materials as those exemplified in the above-described production method of the filament. Among them, as the vitrigel dried body constituting the filament according to the present embodiment, an atelocollagen vitrigel dried body is particularly preferable because it is a biocompatible material having an epithelialization promoting effect and a scar formation inhibiting effect in a wound part.

### <Hydrophobic solvent>

A hydrophobic solvent is adhered to or impregnated into the filament according to the present embodiment.

Examples of the hydrophobic solvent include the same solvents as those exemplified in the above-described production method of the filament. Among these, the hydrophobic solvent contained in the filament according to the present embodiment is preferably a silicone oil.

### <Application>

As will be described in examples below, the filament according to the present embodiment is useful as a suture filament from the viewpoint of that it has the excellent strength and the strength is maintained even in the presence of a body fluid or the like. Further, in a case where the filament according to the present embodiment is used as the suture filament, tissue regeneration is promoted in the wound, and scar is not left. That is, the filament according to the present embodiment is useful as a tissue regeneration filament that can be used as a suture filament.

In particular, in a case where the filament according to the present embodiment is formed of an atelocollagen vitrigel dried body, it has the epithelization promoting effect and the scar formation inhibiting effect in the wound part, and thus it is suitable as a tissue regeneration filament.

In the present specification, the "tissue regeneration filament" means a filament that promotes tissue regeneration at the suture part. Here, "promotion of regeneration of tissue" specifically refers to promotion of wound contraction in the healing process of the wound part, inhibition of granulation tissue formation, and promotion of regenerative epithelial formation by proliferation of epidermal cells.

Moreover, as will be described in examples below, the filament according to the present embodiment has the excellent cell adhesiveness and cell proliferation property. Thus, desired cells can be cultured using the filament according to the present embodiment, and the filament with the cells adhered thereto can be transplanted into a living body of a subject. That is, the filament according to the present embodiment is useful as a cell transplant carrier.

Moreover, as will be described in examples below, the filament of this embodiment is useful as a supplementary material of conjunctival fistula to be used for inserting a treatment device into the vitreous body during a vitreous surgery.

In addition, as will be described in examples below, in a case where the filament according to the present embodiment is formed of an atelocollagen vitrigel dried body, it has an inhibitory action on inflammation and fibrosis of the parietal peritoneum and the visceral peritoneum. Furthermore, it has an inhibitory action on the adhesion between the visceral peritoneums (intestinal tracts). In addition, the filament according to the present embodiment is safe even if it is placed in a living body, and similarly, tissues other than the peritoneum are considered to have an inflammation-inhibiting action or a fibrosis-inhibiting action. That is, the filament according to the present embodiment is useful as an inflammation inhibitor or a fibrosis inhibitor (particularly, peritonitis inhibitor or peritoneal fibrosis inhibitor). In addition, the filament according to the present embodiment is useful as an intestinal adhesion inhibitor.

### EXAMPLES

Hereinafter, the present invention will be described with reference to examples, but the present invention is not limited to the following examples.

### [Production Example 1] Production of filament

### (1) Preparation of columnar atelocollagen gel

First, a 0.5% porcine-derived atelocollagen solution was prepared by mixing equal amounts of 1.0% porcine atelocollagen solution (prepared by Kanto Chemical Co., Inc.) and serum-free culture medium. Subsequently, 1.4 mL of the prepared atelocollagen solution was suctioned into a 1 mL pipette and 7 mL into a 5 mL pipette, respectively. Next, both ends of each pipette were sealed with parafilm, allowed to stand in a cell culture incubator (37°C, 5% CO₂) for 2 hours, and gelled to prepare a columnar atelocollagen gel.

### (2) Irradiation of columnar atelocollagen gel with ultraviolet rays

Next, the parafilm was then removed from both ends of each pipette. The columnar atelocollagen gel was taken out from the inside of the pipette and allowed to stand in a state of being stretched linearly on a flat tray on which a vinyl sheet was laid. Subsequently, the columnar atelocollagen gel was irradiated with ultraviolet rays (irradiation energy: 800 mJ/cm²), and was inverted and irradiated with ultraviolet rays again (irradiation energy: 800 mJ/cm²). The total ultraviolet rays irradiation energy was 1,600 mJ/cm².

### (3) Preparation of filamentous atelocollagen gel dried body

Next, one end of the columnar atelocollagen gel irradiated with ultraviolet rays was fixed and suspended at the edge of a clothesline made of a polypropylene resin. Then, in a simple clean bench equipped with a constant temperature and humidity chamber under the conditions of a temperature of 10°C and a humidity of 40%, the suspended columnar atelocollagen gel was vitrified by sufficiently air-drying to prepare a filamentous atelocollagen gel dried body.

### (4) Preparation of filamentous atelocollagen vitrigel

Next, the obtained filamentous atelocollagen gel dried body was rehydrated with PBS to prepare a filamentous atelocollagen vitrigel.

### (5) Preparation of filamentous atelocollagen vitrigel dried body

Next, one end of the filamentous atelocollagen vitrigel was fixed and suspended at the edge of a clothesline made of a polypropylene resin. Then, in a simple clean bench equipped with a constant temperature and humidity chamber under the conditions of a temperature of 10°C and a humidity of 40%, the suspended filamentous atelocollagen gel was vitrified again by sufficiently air-drying to prepare a filamentous atelocollagen vitrigel dried body.

### (6) Irradiation of filamentous atelocollagen vitrigel dried body with ultraviolet rays

Next, the obtained filamentous atelocollagen vitrigel dried body was allowed to stand in a state of being stretched linearly on a flat tray. Subsequently, the filamentous atelocollagen gel was irradiated with ultraviolet rays (irradiation energy: 400 mJ/cm²), and was inverted and irradiated with ultraviolet rays again (irradiation energy: 400 mJ/cm²). The total ultraviolet rays irradiation energy was 800 mJ/cm².

Regarding the average diameter of the obtained filamentous atelocollagen vitrigel dried body after irradiation with ultraviolet rays, those prepared by using 1 mL pipettes (hereinafter, it may be referred to as "filamentous atelocollagen dried body 1") were 148 ± 42 µm and those prepared by using 5 mL pipettes (hereinafter, it may be referred to as "filamentous atelocollagen vitrigel dried body 2") were 444 ± 30 µm.

### [Example 1] Test for checking breaking strength of filamentous atelocollagen vitrigel dried body

The breaking strength of the filamentous atelocollagen vitrigel dried body 1 and the filamentous atelocollagen vitrigel dried body 2 prepared in Production Example 1 was measured.

As a measurement target, the filamentous atelocollagen vitrigel dried body 1 (hereinafter, it may be referred to as "a dried body 1"), a filamentous atelocollagen vitrigel obtained by rehydration of the dried body 1 (hereinafter, it may be referred to as a "rehydrated body 1"), a filamentous atelocollagen vitrigel dried body 1 infiltrated with a silicone oil (hereinafter, it may be referred to as an "infiltrated body 1"), a filamentous atelocollagen vitrigel dried body 2 (hereinafter, it may be referred to as a "dried body 2"), a filamentous atelocollagen vitrigel by rehydration of the dried body 2 (hereinafter, it may be referred to as a "rehydrated body 2"), a filamentous atelocollagen vitrigel dried body 2 infiltrated with a silicone oil (hereinafter, it may be referred to as an "infiltrated body 2") were prepared.

As controls, a polyglactin suture filament VICRYL (registered trademark) (7-0, average diameter of suture filament standard: about 50 to 69 µm) (hereinafter, it may be referred to as "dried body 3"), VICRYL (registered trademark) after rehydration of the dried body 3 (hereinafter, it may be referred to as "rehydrated body 3"), and VICRYL infiltrated with a silicone oil (registered trademark) (hereafter, it may be referred to as "infiltrated body 3") were also prepared.

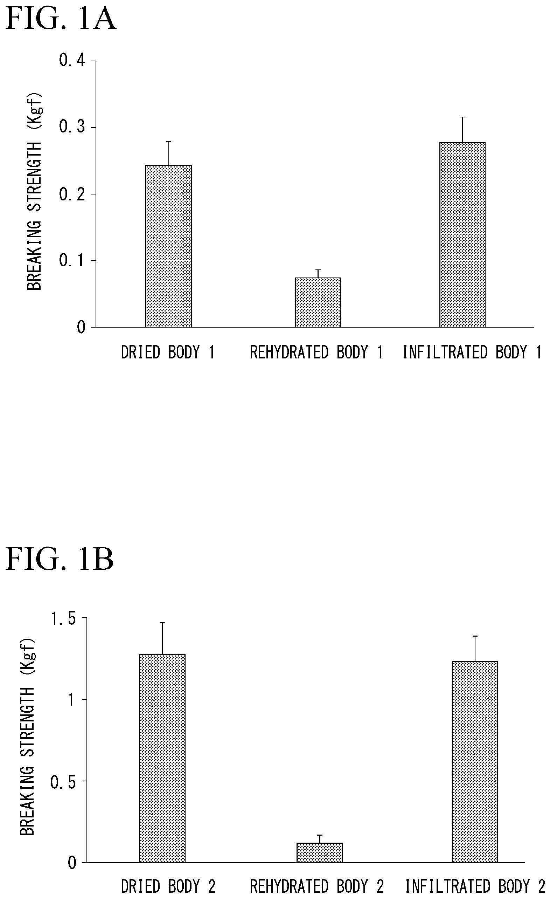

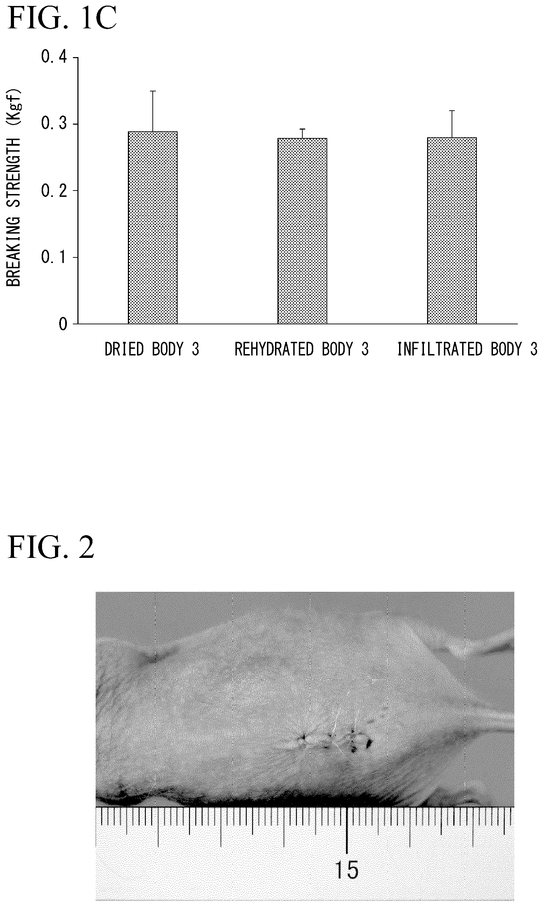

As a specific method of measuring the breaking strength, after cutting the filament to a total length of 5 cm, and the filament pulled at 9 mm per minute while fixing both ends at 1 cm, and the breaking strength (kgf) at this time was measured by using a digital force gauge (manufactured by Nidec-Shimpo Corporation). The results are shown in FIGS. 1A, 1B, and 1C.

FIG. 1A shows the breaking strength in the filamentous atelocollagen vitrigel dried body 1 under the conditions, FIG. 1B shows the breaking strength in the filamentous atelocollagen vitrigel dried body 2 under the conditions, and FIG. 1C shows the breaking strength in the polyglactin suture filament VICRYL (registered trademark) under the conditions.

From FIGS. 1A to 1C, it was found that the dried body 1 and the infiltrated body 1 had substantially the same breaking strength as that of the dried body 3, the rehydrated body 3, and the infiltrated body 3 of VICRYL (registered trademark) which are the controls. On the other hand, the rehydrated body 1 and the rehydrated body 2 had the breaking strength smaller than 0.1 kgf.

In addition, it was found that the dried body 2 and the infiltrated body 2 had a breaking strength larger than 1 kgf, and had more excellent strength than that of the dried body 3 of VICRYL (registered trademark), the rehydrated body 3, and the infiltrated body 3 which are the controls. This was presumed to originate from the size of the average diameter.

### [Example 2] Suture test of incision of mouse using filamentous atelocollagen vitrigel dried body

In order to check that those obtained by infiltrating the filamentous atelocollagen vitrigel dried body 1 obtained in Production Example 1 with a silicone oil (infiltrated body 1) was strong enough to withstand surgery, a suture test of the incision of the mouse epidermis was performed. FIG. 2 shows an image of the mouse on the day when the suture was performed.

From FIG. 2, it was found that the infiltrated body 1 has sufficient strength as a suture filament and does not cause the wound part to be separated after the operation.

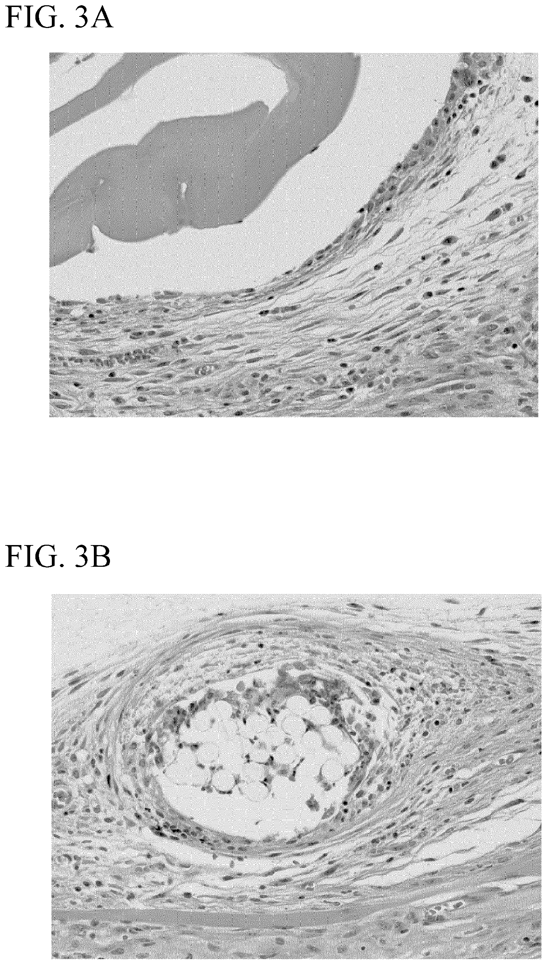

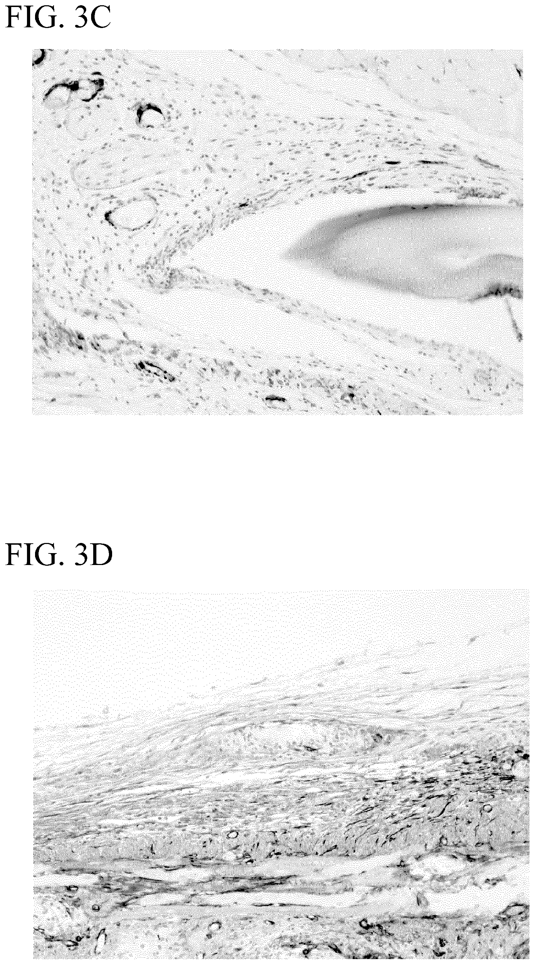

In addition, a tissue section of the suture part on day 7 of suture was prepared, and immunostaining was performed using hematoxylin-eosin (HE) staining and an anti-α smooth muscle actin (a-SMA) antibody. The results of the HE staining are shown in FIG. 3A, and the results of immunostaining with the anti-α-SMA antibody are shown in FIG. 3C,

Further, the suture test of the incision of the mouse epidermis was performed using a polyglactin suture filament VICRYL (registered trademark) (7-0, average diameter of suture filament standard: about 50 to 69 µm) (dried body 3) as a control. As in the case of using the infiltrated body 1, the tissue section of the suture part on 7 of the suture was prepared, and the HE staining and immunostaining using an anti-α-SMA antibody were performed. The results of the HE staining are shown in FIG. 3B, and the results of immunostaining with the anti-α-SMA antibody are shown in FIG. 3D.

From FIG. 3A and FIG. 3B, in the suture part using the infiltrated body 1, similar to the dried body 3, it ligated satisfactorily and the bonding of tissues was found.

Further, from FIG. 3C and FIG. 3D, it was revealed that as compared with the case of using the dried body 3, in a case of using the infiltrated body 1, the number of αSMA-positive myofibroblasts, which are the main constituents of scar formation, is overwhelmingly small around the suture part.

### [Example 3] Supplementing test of porcine conjunctival fistula using filamentous atelocollagen vitrigel dried body

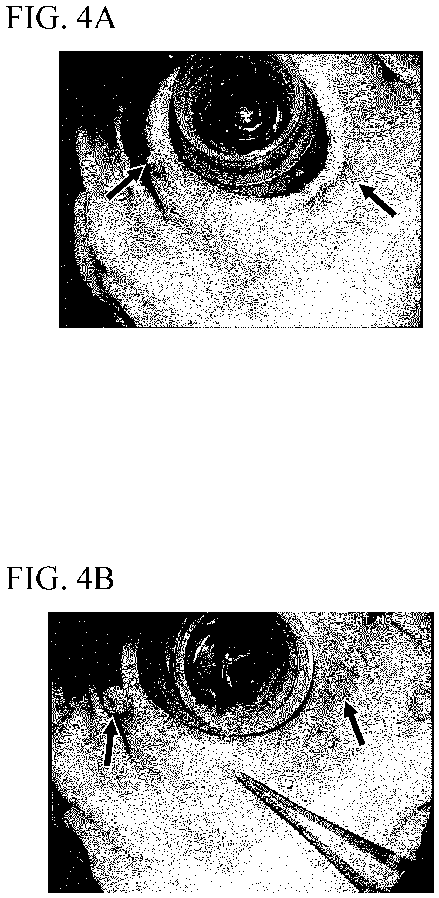

In order to check the excellent strength, the filamentous atelocollagen vitrigel dried body 2 (dried body 2) obtained in Production Example 1 was used as a supplementary material for the porcine conjunctival defect (fistula). FIG. 4A shows an image of the porcine conjunctival fistula on the day embedded as a supplementary material. In FIG. 4A, arrows indicate the embedded dried bodies 2.

Also, as a control, a plastic port that is usually used as a supplementary material for the conjunctival fistula was embedded. The result is shown in FIG. 4B. In FIG. 4B, an arrow indicates embedded plastic ports.

From FIG. 4A and FIG. 4B, it was found that the dried body 2 has excellent strength and can be used as the supplementary material for blocking the conjunctival fistula.

### [Example 4] Test for checking cell adhesion and proliferation using filamentous atelocollagen vitrigel dried body

A cell culture test was performed by using endothelial cells and fibroblasts to check that the one obtained by rehydrating filamentous atelocollagen vitrigel dried body 1 (rehydrated body 1) in Production Example 1 has excellent cell adhesion and proliferation.

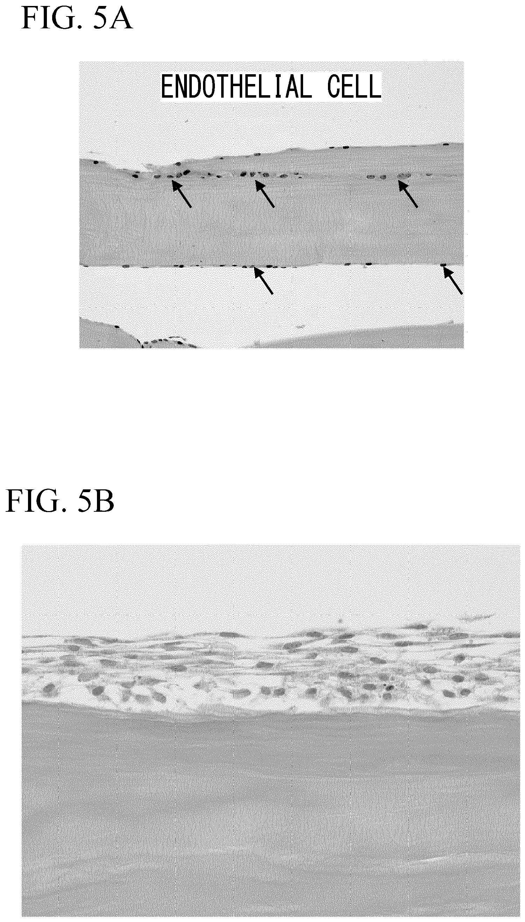

Specifically, the endothelial cells (MS-1, ATCC (American Type Culture Collection)) and the fibroblasts (Wistar rat-derived primary cultured dermal fibroblasts) were each cultured for 10 days in a culture dish with rehydrated body 1 cut into 5 cm. After culturing, the rehydrated body 1 was taken out, stained with HE, and observed using an optical microscope (manufactured by OLYMPUS, BX53). FIG. 5A is an image showing the rehydrated body 1 cultured with the endothelial cells, and FIG. 5B is an image showing the rehydrated body 1 cultured with the fibroblasts. In FIG. 5A, arrows indicate the endothelial cells adhered to the rehydrated body 1.

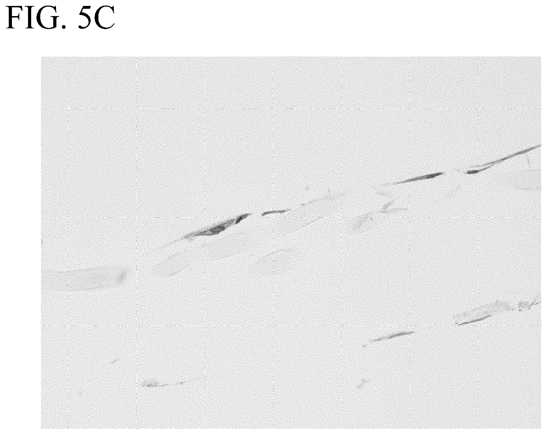

Further, as a control, the one obtained by rehydrating the polyglactin suture filament VICRYL (registered trademark) (rehydrated body 3) in Production Example 1 was, similarly, cut into 5 cm and cultured in a culture dish with fibroblasts for 10 days. After culturing, the rehydrated body 3 was taken out, stained with HE, and observed using an optical microscope (manufactured by OLYMPUS, BX53). FIG. 5C is an image showing the rehydrated body 3 cultured with the fibroblasts.

From FIG. 5C, in the case where the rehydrated polyglactin suture filament VICRYL (registered trademark) was used, only a very small number of cells adhered and the proliferation was low. On the other hand, from FIG. 5B, in a case where the rehydrated filamentous atelocollagen vitrigel was used, a number of cells adhered and proliferated.

In addition, from FIGS. 5A and 5B, it was found that the rehydrated filamentous atelocollagen vitrigel exhibits excellent adhesion and proliferation properties for both endothelial cells and fibroblasts.

### [Example 5] Test for checking peritonitis inhibiting effect and peritoneal fibrosis inhibiting effect of filamentous atelocollagen vitrigel dried body

The effects of placing the filamentous atelocollagen vitrigel dried body 1 and the filamentous atelocollagen vitrigel dried body 2 produced in Production Example 1 on peritoneal fibrosis model mice were examined.

### (1) Insertion and placement of filamentous atelocollagen vitrigel dried body into mouse abdominal cavity

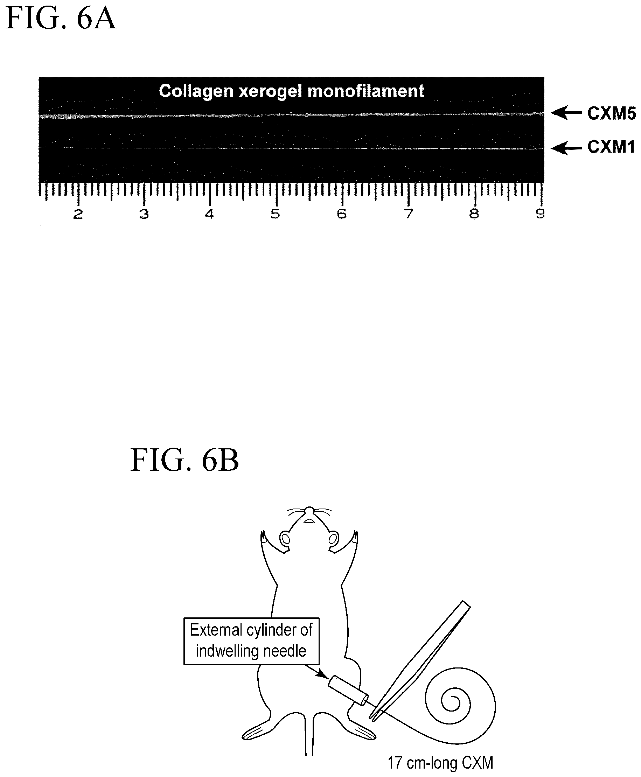

FIG. 6A is an image showing the filamentous atelocollagen vitrigel dried body 1 and the filamentous atelocollagen vitrigel dried body 2 prepared in Production Example 1. In FIG 6A, "CXM 1" indicates the filamentous atelocollagen vitrigel dried body 1, and "CXM 5" indicates the filamentous atelocollagen vitrigel dried body 2. In addition, FIG. 6B is a diagram showing a method of inserting the filamentous atelocollagen vitrigel dried body into the mouse abdominal cavity.

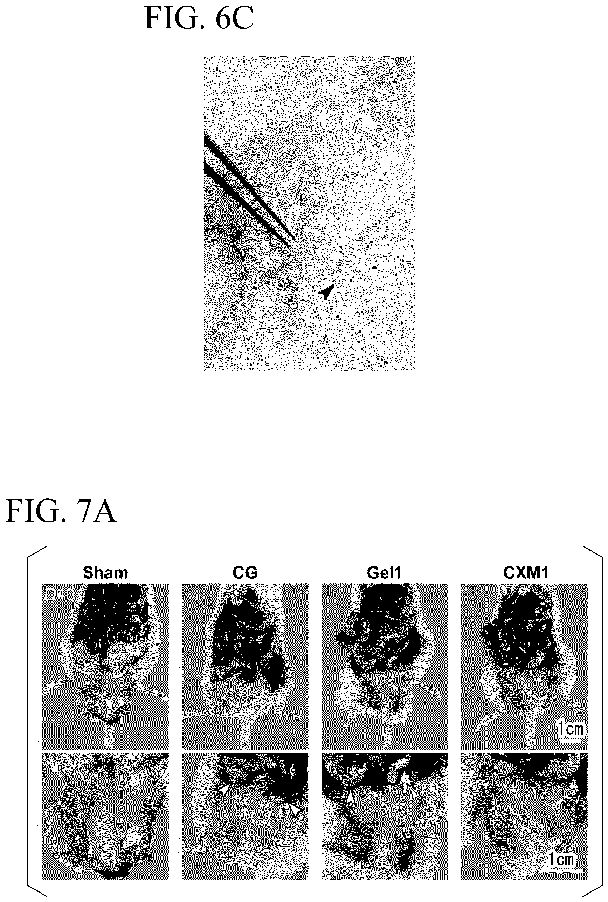

Using an indwelling needle and a tweezer, each of the filamentous atelocollagen vitrigel dried body 1 and the filamentous atelocollagen vitrigel dried body 2 is inserted into the abdominal cavity of a female ICR mouse having a body weight of 40 g (refer to FIG. 6C).

### (2) Induction of peritoneal fibrosis model mouse by intraperitoneal injection of chlorhexidine

On the day after the filamentous atelocollagen vitrigel dried body was placed, a 0.1% chlorhexidine solution (previously prepared using a 15% ethanol-containing physiological saline solution) was administered to the abdominal cavity of the mouse at a dose of 10 mL per kg body weight every other day.

In addition, the following 4 groups including the control test group were prepared.
Sham group: Needle puncture only, no chlorhexidine intraperitoneal injection CG group: Chlorhexidine intraperitoneal injection only
Gel group: Atelocollagen gel intraperitoneal injection, Chlorhexidine intraperitoneal injection
CXM group: Filamentous atelocollagen vitrigel dried body intraperitoneal insertion, Chlorhexidine intraperitoneal injection

### (3) Observation of the peritoneum

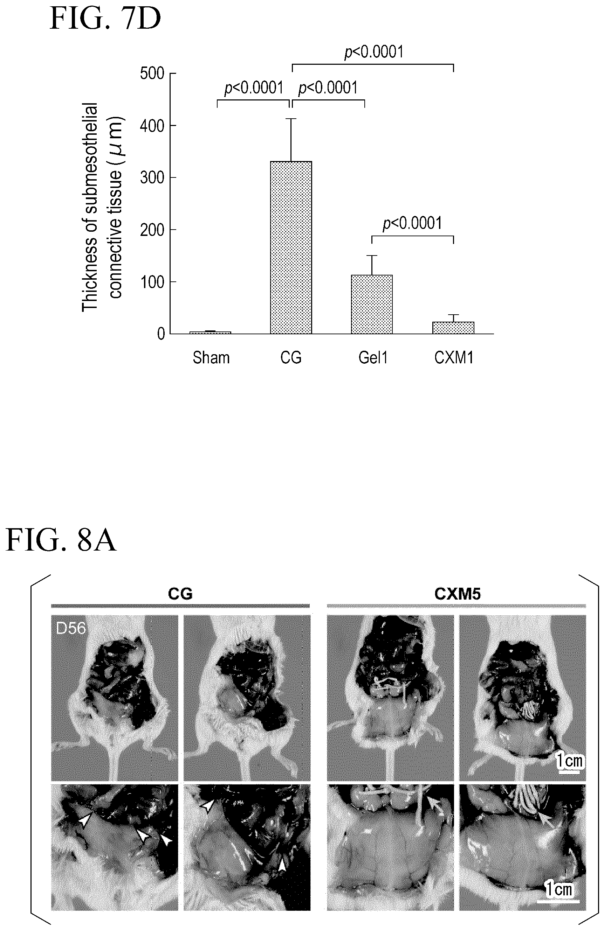

On day 40 and day 56 after the induction of peritonitis, one mouse in each group was cut the abdomen open and observed visually. The results are shown in FIG. 7A (day 40 after the induction of peritonitis) and FIG. 8A (day 56 after induction of peritonitis). In FIG. 7A, the arrows shown in the CG group and the Gel group indicate adhesion between the peritoneum and the intestinal tracts, the arrows shown in the Gel group indicate a collagen gel placed in the peritoneum, and the arrow shown in the CXM group indicates a filamentous atelocollagen vitrigel placed in the peritoneum. In FIG. 8A, the arrows shown in the CG group indicate adhesion between the peritoneum and the intestinal tracts, and the arrow shown in the CXM group indicates a filamentous atelocollagen vitrigel placed in the peritoneum.

### (4) HE staining and Azan staining

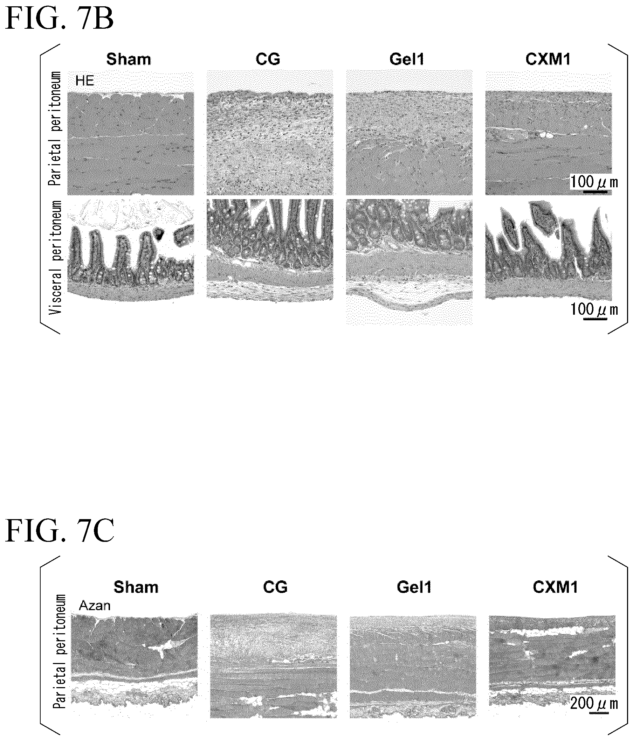

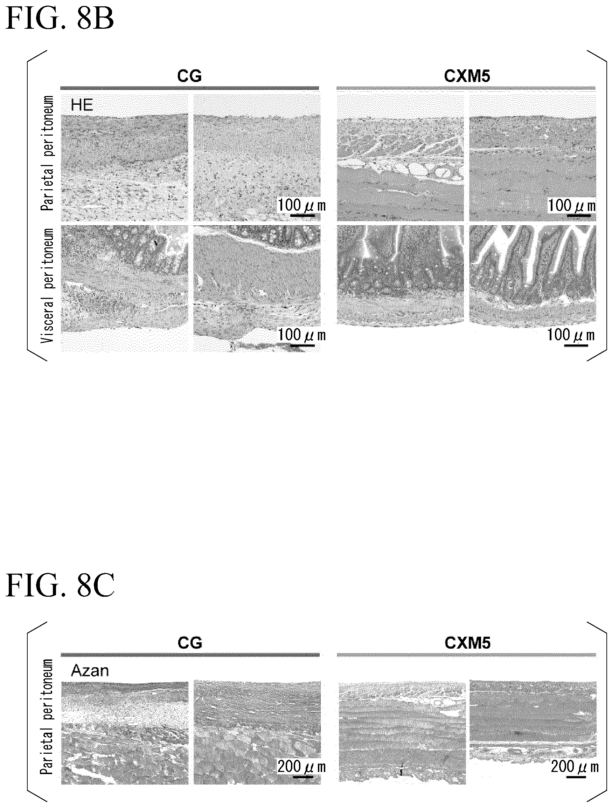

Next, the tissue sections of the peritoneum of each group of mice on day 40 and day 56 after the induction of peritonitis were prepared and HE staining and Azan staining were performed. The results of each group of mice on day 40 after the induction of peritonitis were shown in FIG. 7B (HE stained image) and FIG. 7C (Azan stained image). In addition, the results of each group of mice on day 56 after the induction of peritonitis were shown in FIG. 8B (HE stained image) and FIG. 8C (Azan stained image).

In FIG. 7B and FIG. 8B, the upper images are the HE stained images of the parietal peritoneums, and the lower images are the HE stained images of the visceral peritoneum. The scale bar indicates 100 µm. Moreover, in FIG. 7C and FIG. 8C, a scale bar indicates 200 µm.

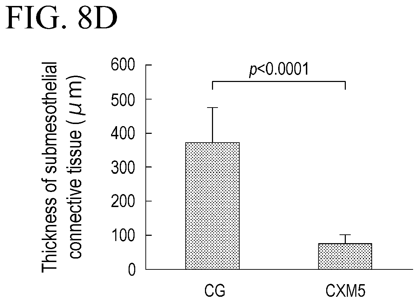

Further, from the Azan stained images shown in FIG. 7C and FIG. 8C, the thickness (µm) of the submesothelial connective tissue of each group of mice was measured, and the average value was shown in a graph (refer to FIG. 7D and FIG. 8D).

From FIG. 7A to FIG. 7D, at day 40 after the induction of peritonitis, the fibrosis of the parietal peritoneum was significantly inhibited in the Gel group and the CMX group as compared with the CG group. In addition, the CMX group had a particularly remarkable effect of inhibiting fibrosis of the parietal peritoneum than the Gel group.

From FIG. 8A to FIG. 8D, at day 56 after the induction of peritonitis, the fibrosis of the parietal peritoneum was significantly inhibited in the CMX group as compared with the CG group. From this point on, the weight loss of the CG group was significant and the test was not able to be continued.

### (5) Immunostaining using antibodies against fibrosis marker

Next, tissue sections of the peritoneum of each group of mice on day 40 after induction of peritonitis were prepared and immunostained with various antibodies. The antibodies used are described below.
anti-cytokeratin AE1/AE3 (CKAE1/AE3) antibody: CKAE1/AE3 is a general-purpose epithelial marker.
Anti-Vimentin antibody: Vimentin is an intermediate filament characteristic of mesenchymal cells. Marker of mesenchymal cells.
Anti-N-cadherin antibody: N-cadherin is expressed in a process in which epithelial cells differentiate into mesenchymal cells.
Marker of epithelial-mesenchymal transition.
Anti-connective tissue growth factor (CTGF) antibody: CTGF is a factor that promotes fibroblast proliferation and collagen production.
Fibrosis marker.
Anti-α-SMA antibody: α-SMA is expressed in a process in which epithelial cells differentiate into mesenchymal cells.
Marker of epithelial-mesenchymal transition.

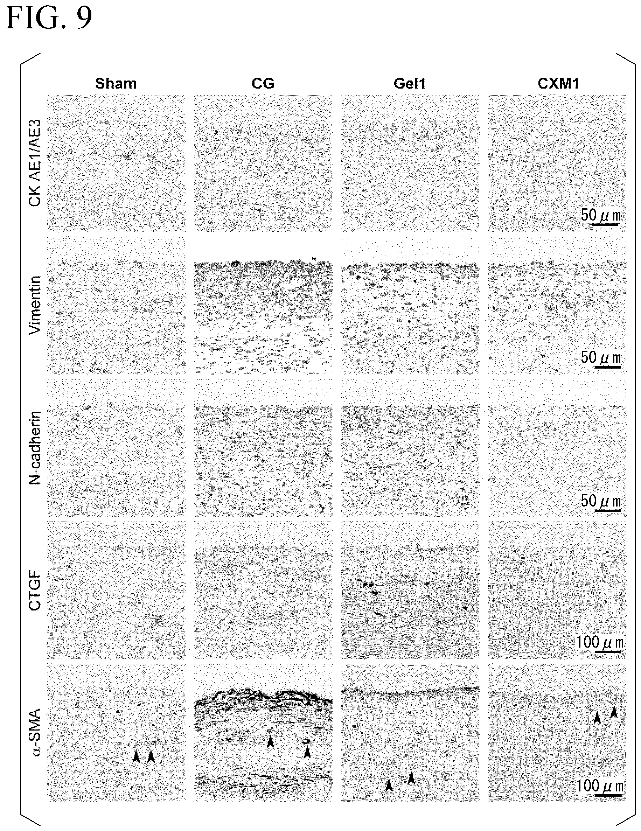

The results of immunostaining are shown in FIG. 9. In FIG. 9, the scale bar of the immunostained image with an anti-cytokeratin CKAE1/AE3 antibody, an anti-vimentin antibody, and an anti-N-cadherin antibody indicates 50 µm. The scale bar of the immunostained image with anti-CTGF antibody and anti-α-SMA indicates 100 µm. In the immunostained image with the anti-α-SMA antibody in FIG. 9, the arrows indicate a blood vessel wall serving as a positive control.

From FIG. 9, at day 40 after the induction of peritonitis, the CXM group inhibited the appearance of vimentin positive cells, N-cadherin positive cells, and α-SMA positive cells as compared to the CG group. This means that the epithelial-mesenchymal transition of mesothelial cells and the appearance of myofibroblasts, which cause peritoneal fibrosis, were inhibited.

### (6) Immunostaining using antibodies against inflammatory marker

Next, tissue sections of the peritoneum of each group of mice on day 40 after induction of peritonitis were prepared and immunostained with various antibodies. The antibodies used are described below.
Anti-CD45 antibody: CD45 is a marker for leukocytes.
Anti-F4/80 antibody: F4/80 is a marker for mouse mature macrophages.
Anti-proliferating cell nuclear antigen (PCNA) antibody: PCNA is a cell cycle-related nuclear protein.
Markers of cell proliferation and cell cycle (G1 to S cycles)

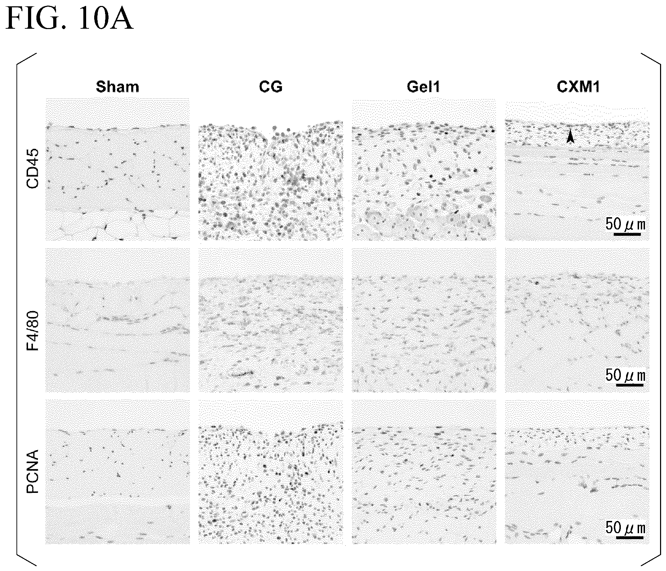

The results of immunostaining are shown in FIG. 10A. In FIG. 10A, the scale bar indicates 50 µm.

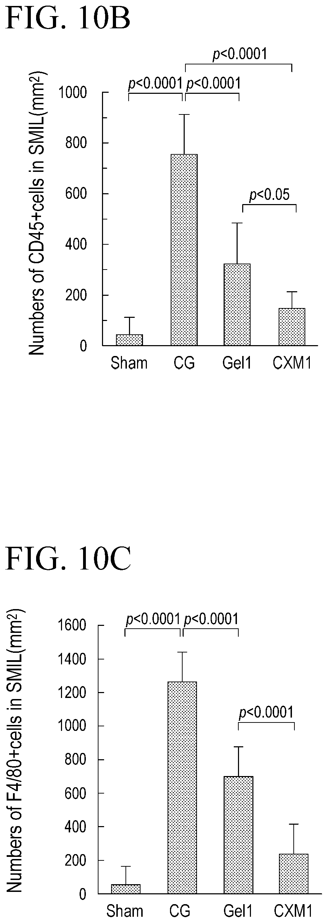

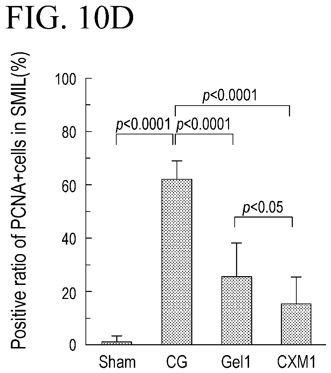

Further, from each immunostained image of FIG. 10A, the number of CD45 positive cells, the number of F4/80 positive cells, and the proportion of PCNA positive cells in the submesothelial interstitial layer (SMIL) of mice were calculated and shown in the graph (refer to FIGS. 10B to 10D). Note that, the number of each positive cell is expressed in area (mm²) in FIGS. 10B and 10C, and is represented by the ratio (%) of the area of a PCNA positive cell with respect to the entire area of the SMIL in FIG. 10D.

From FIG. 10A to FIG. 10D, at the time of day 40 after the induction of peritonitis, in the CXM group, the appearance of CD45-positive leukocytes, F4/80-positive macrophages, and PCNA-positive mesenchymal cells was significantly inhibited as compared to the CG group. From these results, it was suggested that peritoneal fibrosis was inhibited by inhibiting peritonitis in the CMX group.

From the above, it was found that the filamentous atelocollagen vitrigel dried body inhibited the peritoneal inflammation and fibrosis caused by chlorhexidine. The collagen in a gel state exhibited similar peritoneal inflammation inhibiting effect and fibrosis inhibiting effect, but a significant difference was observed between the filamentous atelocollagen vitrigel dried body and the collagen in a gel state. That is, the filamentous atelocollagen vitrigel dried body had significantly superior peritoneal inflammation inhibiting effect and fibrosis inhibiting effect than the collagen in a gel state.

In addition, even on day 56 after the induction of peritonitis, no significant change was observed in the properties of the filamentous atelocollagen vitrigel dried body (refer to FIGS. 8A to 8C). From this, it was speculated that the peritoneal fibrosis inhibiting effect is continue after this.

### Industrial Applicability

According to the production method of the filament according to the present embodiment, it is also possible to produce a filament having excellent strength. In addition, in a case where the filament obtained by the above production method is formed of the atelocollagen vitrigel dried body, the atelocollagen vitrigel dried body is converged with high density, and has an epithelization promoting effect and a scar formation inhibiting effect. Therefore, the filament is useful as a tissue regeneration filament. Further, the filament obtained by the above production method has excellent cell adhesion and proliferation. For this reason, it is useful as a cell transplant carrier. In addition, the filament obtained by the above production method has an inhibitory action on inflammation and fibrosis of the parietal peritoneum and visceral peritoneum. Furthermore, it has an inhibitory action on the adhesion between the visceral peritoneums (intestinal tracts). For this reason, it is useful as a peritonitis inhibitor, a peritoneal fibrosis inhibitor, or an intestinal adhesion inhibitor.

## Claims

1. A production method of a filament, comprising in this order:
a step A of irradiating columnar hydrogel with ultraviolet rays;
a step B of vitrifying the columnar hydrogel after irradiation with the ultraviolet rays to obtain a filamentous hydrogel dried body; and
a step C of rehydrating the filamentous hydrogel dried body to obtain a filamentous vitrigel.

2. The production method of the filament according to Claim 1, further comprising:
a step D of re-vitrifying the filamentous vitrigel to obtain a filamentous vitrigel dried body after the step C.

3. The production method of the filament according to Claim 1 or 2, further comprising:
a step E of re-irradiating the filamentous vitrigel dried body with the ultraviolet rays after the step D.

4. The production method of the filament according to any one of Claims 1 to 3, further comprising:
a step F of adhering or impregnating a hydrophobic solvent to the filamentous vitrigel dried body re-irradiated with the ultraviolet rays after the step E.

5. The production method of the filament according to any one of Claims 1 to 4,
wherein the hydrogel is an atelocollagen gel.

6. The production method of the filament according to any one of Claims 1 to 5,
wherein the filament is a tissue regeneration filament usable as a suture filament.

7. The production method of the filament according to any one of Claims 1 to 5,
wherein the filament is a cell transplant carrier.

8. The production method of the filament according to any one of Claims 1 to 7,
wherein the filament is a supplementary material of a conjunctival fistula.

9. The production method of the filament according to any one of Claims 1 to 5, wherein the filament is a peritonitis inhibitor or a peritoneal fibrosis inhibitor.

10. A filament which is formed of a vitrigel dried body, and to which a hydrophobic solvent is adhered or impregnated.

11. The filament according to Claim 10,
wherein a breaking strength of the filament is 0.1 kgf or more.

12. The filament according to Claim 10 or 11,
wherein a breaking strength of the filament is 0.2 kgf or more.

13. The filament according to any one of Claims 10 to 12,
wherein the vitrigel dried body is an atelocollagen vitrigel dried body.

14. The filament according to any one of Claims 10 to 13,
wherein the hydrophobic solvent is a silicone oil.

15. The filament according to any one of Claims 10 to 14,
wherein the filament is a tissue regeneration filament usable as a suture filament.

16. The filament according to any one of Claims 10 to 14,
wherein the filament is a cell transplant carrier.

17. The filament according to any one of Claims 10 to 16,
wherein the filament is a supplementary material of a conjunctival fistula.

18. The filament according to any one of Claims 10 to 14,
wherein the filament is a peritonitis inhibitor or a peritoneal fibrosis inhibitor.
